# DEMANDE DE BREVET EUROPEEN

(11) **EP 1 216 623 A1**
(43) Date de publication de la demande: **26.06.2002**
(21) Numéro de dépôt: 01402878.1
(22) Date de dépôt: 08.11.2001
(51) Int. Cl.: A23L 1/30, A23L 1/302, A23L 1/304, A61K 7/48

(54) **Compositions diététiques et/ou cosmétiques pour améliorer la sécheresse des muqueuses**

(30) Priorité: 08.11.2000 FR 0014336
(71) Demandeur: BRIF, 75020 Paris (FR)
(72) Inventeur: Correard, Brigitte, 75020 Paris (FR); Correard, Stéphane, 75011 Paris (FR)
(74) Mandataire: Burtin, Jean-François

(57) **Abrégé**

L'invention se rapporte au domaine de la diététique et de la cosmétique.

Elle a spécifiquement pour objet des compositions cosmétiques et/ou diététiques destinées à la voie orale caractérisées en ce qu'elles sont formées :
- d'un ou plusieurs agents précurseurs de la synthèse des prostaglandines
- d'un ou plusieurs agents anti-oxydants
- d'un ou plusieurs micro-éléments stabilisant les synthèses organiques
- et éventuellement d'un ou plusieurs adoucissants, en association ou en mélange avec un ou plusieurs excipients ou diluants inertes non toxiques.

Utilisation pour remédier aux inconvénients de la sécheresse des muqueuses.

## Description

L'invention s'adresse au domaine de la diététique et de la cosmétique.

L'invention concerne plus particulièrement des compositions diététiques sous forme de compléments alimentaires pour remédier aux inconvénients de la sécheresse des muqueuses.

L'invention a spécifiquement pour objet des compositions cosmétiques et/ou diététiques destinées à la voie orale caractérisées en ce qu'elles sont formées :
- d'un ou plusieurs agents précurseurs de la synthèse des prostaglandines,
- d'un ou plusieurs agents antioxydants,
- d'un ou plusieurs microéléments stabilisant les synthèses organiques,
- et éventuellement d'un ou plusieurs agents adoucissants intervenant pour conférer de l'élasticité aux différentes muqueuses,
en association ou en mélange avec un ou plusieurs excipients ou diluants inertes, non toxiques, pharmaceutiquement acceptables.

On connaît à l'heure actuelle les effets du stress et plus particulièrement des substances oxydantes sur l'état d'hydratation des différentes muqueuses de l'organisme et spécialement des muqueuses en contact avec l'atmosphère et le milieu environnant.

C'est ainsi que la muqueuse ophtalmique ou la muqueuse nasale sont particulièrement sensibles à la présence d'un film continu à base de mucopolysaccharides qui protège ou qui humidifie en permanence ces conduits ouverts sur l'extérieur. De la même façon la sécheresse du milieu aqueux de l'oeil intervient pour déclencher ce que l'on appelle le syndrome de l'oeil sec accompagné de sensations désagréables dues à la formation de petits cristaux ou de corps étrangers à la surface du globe oculaire.

Pour cette même raison la gorge a besoin d'être protégée en permanence par un film de substances hydratantes qui évitent la contamination microbienne, les enrouements ou les difficultés de déglutition.

Il en est de même pour l'apparence de la peau qui a besoin d'être hydratée en permanence soit par la voie externe soit par la voie générale.

Ces troubles bénins peuvent être traités par des médications appropriées mais il s'avère être peu utile d'employer des agents thérapeutiques énergiques dont les effets secondaires peuvent être nombreux sans apport thérapeutique réel ou permanent.

Dans ces conditions, l'apport d'un supplément alimentaire qui est destiné à compenser les effets induits par une déficience dans les secrétions externes protectrices des muqueuses constitue un avantage certain sur le plan de l'efficacité et une grande commodité du fait de l'innocuité totale des compléments alimentaires mis en oeuvre pour la réalisation de la présente invention.

Parmi les constituants des compositions selon l'invention on citera en particulier les acides gras mono- ou polyinsaturés qui sont les précurseurs normaux des prostaglandines et notamment de la prostaglandine E1 dont les propriétés tonifiantes sur la peau et les muqueuses sont bien connues. On pourra citer à cet égard l'huile d'onagre ou huile d'evening primrose, l'huile de saumon, l'huile de bourrache, l'huile de pépins de cassis, l'huile de noisettes, les huiles de poisson mais aussi des acides gras à des degrés de pureté variables pouvant contenir les acides gras sous forme libre ou sous forme de mono, de di ou de triglycérides ou bien encore pouvant engager ces acides gras sous forme de combinaisons chimiques telles que des lécithines, des phospholécithines, des sphingomyélines... et similaires.

Parmi les agents antioxydants utilisés dans les compositions selon l'invention on pourra distinguer des dérivés non saturés comme l'acide ascorbique, l'acide isoascorbique, les sels de métal alcalin, ou alcalins-terreux de l'acide ascorbique, des esters d'acide gras d'acide ascorbique... On pourra également utiliser des dérivés thiols comme la cystéine, l'acétylcystéine, la methionine, le ∝-thioglycérol ou l'acide mercapto éthanesulfonique. On pourra citer encore d'autres agents antioxydants comme les vitamines du groupe B (vitamine B 2, vitamine B 6, vitamine PP et vitamine B 12). Comme agents antioxydants on pourra citer également des phénols sous forme libre ou glucosilée extraits de plantes, de fleurs ou de fruits. Parmi ceux-ci on pourra citer l'arbutoside, les flavones, les isoflavones naturelles ou synthétiques ou des molécules polyphénoliques comme l'acide gallique, l'acide catechique, l'acide vanillyl mandelylique...

Les microéléments qui entrent dans la composition des préparations selon l'invention sont ceux qui sont utiles ou qui participent à des réactions enzymatiques comme apoenzymes ou comme co-enzymes. C'est le cas de sels de zinc, de cuivre, de cobalt, de manganèse, de dérivés minéraux ou organiques du sélénium.

Une forme particulièrement utilisable de composés contenant des microéléments physiologiques est la levure de bière qui peut être cultivée sur un milieu enrichi en de tels microéléments. Dans ces conditions la teneur en silicium, en sélénium, en germanium, en cuivre, en zinc ou en cobalt pourra être garantie.

Les compositions selon l'invention sont préparées selon les méthodes habituelles de la pharmacotechnie dans lesquelles on incorpore graduellement un concentré de principes actifs préparé à l'avance à un ou plusieurs diluants ou excipients jusqu'à réalisation d'une masse parfaitement homogène, puis que l'on tamise pour obtenir une poudre d'une finesse et d'une fluidité appropriée pour permettre la compression en comprimés ou en noyaux de dragées, en sachets de poudre, en granulés aromatisés ou non, ou bien encore la répartition de la poudre en gélules après adjonction d'un agent lubrifiant.

Les compositions diététiques selon l'invention trouvent un emploi pour compenser les inconvénients ou l'inconfort lié à une insuffisance de secrétions telles que lacrymales, nasales, buccales, pharyngées ou encore vaginales.

Les compositions selon l'invention assurent rapidement le retour à une situation normale.

On trouvera ci-après deux exemples de réalisation de l'invention qui ne présentent évidemment aucun caractère limitatif.

### EXEMPLE I

### Capsules antidessèchement

- huile d'onagre naturelle 350 mg
- Huile de saumon naturelle 100 mg
- Vitamine C 60 mg
- Levure de sélénium 25 mg
- Béta-carotène naturel 13 mg
- Extrait de calendula 12 mg
- Vitamine E 7,5 mg
- Sulfate de zinc mono hydraté 7,5 mg
- Vitamine B 6 1 mg
- Vitamine B12 sous forme de cyanocobalamine 0,5 mg
- Excipents de charge dont notamment de l'huile de soja 73,5 mg

### EXEMPLE II

### Capsules pour les muqueuses

- Huile d'onagre naturelle de première pression 200 mg
- Huile de chair de saumon naturelle 200 mg
- Vitamine C 60 mg
- Levure de sélénium naturelle (Se 2 mg/g) 35 mg
- Béta-carotène naturel (poudre d'algues) 20 mg
- Extrait de calendula naturel 12 mg
- Extrait de mauve 12 mg
- Vitamine E (∝-tocophérol à 67 %) 7,5 mg
- Vitamine B 6 (chlorhydrate de pyridoxine) 1 mg
- Vitamine 12 (cyanocobalamine) 0,5 mg
- Substances auxiliaires :
- Cire d'abeille }
- Huile de palme } 52 mg
- Huile de coprah }

## Revendications

1. Nouvelles compositions diététiques et/ou cosmétiques destinées à remédier aux inconvénients de la sécheresse des muqueuses **caractérisées en ce qu'**elles sont formées
- d'un ou plusieurs agents précurseurs de la synthèse des prostaglandines
- d'un ou plusieurs agents anti-oxydants
- d'un ou plusieurs microéléments stabilisant les synthèses organiques
- et éventuellement d'un ou plusieurs agents adoucissants
en association ou en mélange avec un ou plusieurs excipients ou diluants inertes non toxiques adaptés à la voie orale.

2. Nouvelles compositions diététiques et/ou cosmétiques selon la revendication 1, dans lesquelles les agents précurseurs de la synthèse des prostaglandines sont constitués par des acides gras mono ou poly-insaturés sous forme libre ou sous forme de glycérides.

3. Compositions diététiques et/ou cosmétiques selon la revendication 1 et la revendication 2 dans lesquelles les glycérides d'acide gras mono ou poly-insaturé sont des mono, des di ou des triglycérides.

4. Compositions diététiques et/ou cosmétiques selon l'une des revendications précédentes dans lesquelles les précurseurs de la synthèse des prostaglandines sont sous la forme d'une huile choisie parmi l'huile d'onagre ou l'huile d'evening primerose, l'huile de saumon, l'huile de noisettes et les huiles de poisson.

5. Compositions diététiques et/ou cosmétiques selon l'une des revendications précédentes dans lesquelles les agents anti-oxydants sont choisis parmi les produits non saturés, les dérivés thiols, les vitamines du groupe B et les phénols sous forme libre ou glucosidée.

6. Compositions diététiques et/ou cosmétiques selon la revendication 5, dans lesquelles les dérivés non saturés sont choisis parmi l'acide ascorbique, l'acide isoascorbique, les sels de métal alcalin ou alcalinoterreux de l'acide ascorbique et les esters d'acide gras d'acide ascorbique.

7. Compositions diététiques et/ou cosmétiques selon la revendication 5, dans lesquelles les composés thiols sont choisis parmi la cystéine, l'acétylcystéine, la méthionine, l'∝-thioglycérol et l'acide mercapto-éthanesulfonique.

8. Compositions diététiques et/ou cosmétiques selon la revendication 5, dans lesquelles l'agent anti-oxydant est choisi parmi la vitamine B 2 , la vitamine B 6 , la vitamine PP et la vitamine B 12.

9. Compositions diététiques et/ ou cosmétiques selon la revendication 5, dans lesquelles l'agent anti-oxydant est un phénol extrait de plantes, de fleurs ou de fruits ou un composé polyphénolique.

10. Compositions diététiques et/ou cosmétiques selon la revendication 1, dans lesquelles les micro-éléments sont des composés utiles ou qui participent à des réactions enzymatiques.

11. Compositions diététiques et/ou cosmétiques selon la revendication 10, dans lesquelles les micro-éléments sont choisis parmi les sels de zinc, de cuivre, de cobalt, de manganèse et les dérivés minéraux ou organiques du selenium.

12. Compositions diététiques et/ou cosmétiques selon la revendication 10 ou la revendication 11, dans lesquelles le composé contenant des micro-éléments est la levure de bière enrichie en de tels micro-éléments.

13. Procédé de préparation des compositions diététiques et/ou cosmétiques selon l'une des revendications 1 à 12, **caractérisé en ce qu'**on incorpore graduellement un concentré de principes actifs préparé à l'avance à un ou plusieurs diluants ou excipients jusqu'à homogénéité parfaite, tamise le mélange et répartit en comprimés, dragées, poudres, granulés ou en gélules.
